# EUROPEAN PATENT APPLICATION

(11) **EP 4 837 569 A1**
(43) Date of publication of application: **11.11.2026**
(21) Application number: 25736265.7
(22) Date of filing: 02.01.2025
(51) Int. Cl.: A61K 31/196, A61K 45/06, A61P 25/00, A61F 7/00

(54) **COMPOSITION AND METHOD FOR REDUCING BRAIN DAMAGE, DISABILITY, AND/OR DEATH BY COMBINED THERAPY WITH TETRAFLUOROBENZYL DERIVATIVE AND HYPOTHERMIA**

(30) Priority: 02.01.2024 KR 20240000132
(71) Applicant: GNT Pharma Co., Ltd., Yongin-si, Gyeonggi-do 17096 (KR)
(72) Inventor: GWAG, Byoung Joo, Yongin-si, Gyeonggi-do 16951 (KR); AN, Chun San, Suwon-si, Gyeonggi-do 16682 (KR); LEE, Jin Hwan, Hwaseong-si, Gyeonggi-do 18505 (KR)
(74) Representative: Delbarba, Andrea
(86) International application number: PCT/KR2025/000062
(87) International publication number: WO 2025/147111

(57) **Abstract**

The present invention relates to a pharmaceutical use, of a compound of formula 1 or a salt thereof, for improving mortality, neurological impairment, and/or daily activity in patients with resuscitated cardiac arrest receiving target temperature management (TTM) for maintaining a temperature between about 32°C and about 36°C, patients with ischemic or hemorrhagic stroke receiving TTM, patients with traumatic brain or spinal cord injury receiving TTM, or patients with hypoxic-ischemic encephalopathy receiving TTM.

## Description

### TECHNICAL FIELD

The present application claims priority based on Korean Patent Application No. 10-2024-0000132 filed on January 2, 2024, and all contents disclosed in the specification and drawings of said application are incorporated by reference into the present application.

The present invention relates to a method, medical use, and a pharmaceutical composition for improving the mortality or condition of resuscitated cardiac arrest patients, patients with ischemic or hemorrhagic stroke, patients with traumatic brain or spinal cord injury, and patients with hypoxic-ischemic encephalopathy (infants), receiving TTM.

### BACKGROUND ART

Cardiac arrest occurs when the heart suddenly stops beating and is a leading cause of death and disability worldwide. It is reported that approximately 3.8 million people globally experience out-of-hospital cardiac arrest (OHCA) every year. In the United States and Europe, the number of patients experiencing OHCA and in-hospital cardiac arrest (IHCA) annually is estimated at 600,000 and about 300,000, respectively. Currently, the mortality rate for cardiac arrest patients is very high; in fact, about 10% of OHCA and 25% of IHCA patients survive until discharge. Cardiac arrest patients who are successfully resuscitated suffer fatal brain damage following total ischemia-reperfusion, leading to disability and death.

Currently, target temperature management (TTM) is being presented as a guideline for treating resuscitated cardiac arrest patients. It is known that TTM alleviates primary ischemic brain injury and secondary reperfusion brain injury by reducing mitochondrial damage, reactive oxygen species toxicity, excitatory glutamate toxicity, and brain metabolism (Hosseini, Wilson et al. (2020). "Resuscitating the Globally Ischemic Brain: TTM and Beyond." Neurotherapeutics 17(2): 539-562.).

More than 40% of resuscitated cardiac arrest patients experience hyperthermia within 48 hours. This hyperthermia further increases cerebral metabolism, makes neurons more sensitive to excitotoxicity, and damage to the blood-brain barrier leads to neuronal damage and neurological dysfunction in cardiac arrest patients (Sekhon, Ainslie et al. (2017). "Clinical pathophysiology of hypoxic ischemic brain injury after cardiac arrest: a "two-hit" model." Crit Care 21(1): 90.). Hypothermia treatment reduces brain injury following ischemia-reperfusion through various mechanisms, including regulation of brain metabolic rates, control of inflammation and the toxicity of the excitatory neurotransmitter glutamate, and protection of the blood-brain barrier (Yenari, M. A. and H. S. Han (2012). "Neuroprotective mechanisms of hypothermia in brain ischemia." Nat Rev Neurosci 13(4): 267-278.). It was proven for the first time through clinical trials that TTM treatment improves disability over the long term in patients with cardiac arrest due to ventricular fibrillation (Bernard, Gray et al. 2002, Hypothermia after Cardiac Arrest Study 2002). However, in a large-scale European clinical trial involving 939 cardiac arrest patients who fell into a coma after spontaneous circulation, the group treated with hypothermia at 33°C did not show improvement in neurological function compared to the normal body temperature group (Nielsen, N. et al., (2013). "Targeted temperature management at 33 degrees C versus 36 degrees C after cardiac arrest." N Engl J Med 369(23): 2197-2206.). Meanwhile, in a recent clinical trial involving comatose cardiac arrest patients successfully resuscitated with a non-shockable rhythm, patients who received hypothermia treatment at 33°C for 24 hours showed improved neurological function and higher survival rates after 90 days compared to normal body temperature (Lascarrou, J. B. et al., (2019). "Targeted Temperature Management for Cardiac Arrest with Nonshockable Rhythm." N Engl J Med 381(24): 2327-2337.).

Currently, the American Heart Association (AHA), the International Resuscitation Coordination Committee (ILCOR), the European Resuscitation Council (ERC), and the Korean Association of Cardiopulmonary Resuscitation recommend TTM therapy for the treatment of post-cardiac arrest syndrome (PCAS) in OHCA and IHCA patients, but the effects of TTM on improving neurological function and survival are limited. Therefore, active development of drugs that protect brain cells in resuscitated cardiac arrest patients with TTM is underway to provide more efficient treatment for PCAS.

In the brains of cardiac arrest patients successfully resuscitated by the resumption of spontaneous circulation, the excessive release and accumulation of glutamate leads to the overactivation of NMDA receptors, and the influx and overload of calcium ions into neurons cause rapid neuronal death (Won, S. J. et al., (2002). "Cellular and molecular pathways of ischemic neuronal death." J Biochem Mol Biol 35(1): 67-86.). However, the administration of memantine, an NMDA receptor antagonist approved for the treatment of Alzheimer's disease, did not demonstrate a neuroprotective effect in a porcine model of cardiac arrest treated with hypothermia (Rimpilainen, J. et al., (2001). "The N-methyl-D-aspartate antagonist memantine has no neuroprotective effect during hypothermic circulatory arrest: a study in the chronic porcine model." J Thorac Cardiovasc Surg 121(5): 957-968; discussion 968-970; Lipton, S. A. (2004). "Paradigm shift in NMDA receptor antagonist drug development: molecular mechanism of uncompetitive inhibition by memantine in the treatment of Alzheimer's disease and other neurologic disorders." J Alzheimers Dis 6(6 Suppl): S61-74.).

The efficacy of drugs that regulate glutamate neurotoxicity, oxidative stress, and inflammation is being evaluated to prevent brain damage following ischemia-reperfusion in resuscitated cardiac arrest patients. However, it has been confirmed that these investigational drugs do not necessarily exert an effect in TTM treatment. In a Phase 2 clinical trial involving 110 OHCA patients who successfully resuscitated and received TTM treatment, patients administered xenon gas, an NMDA receptor antagonist, showed a significant reduction in white matter (neural network) damage, but no improvement in neurological function was observed after 6 months (Laitio, R. et al., (2016). "Effect of Inhaled Xenon on Cerebral White Matter Damage in Comatose Survivors of Out-of-Hospital Cardiac Arrest: A Randomized Clinical Trial." JAMA 315(11): 1120-1128.). Clinical trials are underway to confirm the efficacy and safety of antioxidants such as high-dose vitamin C and molecular hydrogen (H₂) in resuscitated cardiac arrest patients, and clinical trials are also underway to confirm the inflammatory biomarkers and neurological function improvement effects of the anti-inflammatory drug methylprednisolone in 120 resuscitated comatose cardiac arrest patients, but no effective treatment has been developed yet.

### DISCLOSURE

### Technical Problem

Accordingly, the object that the present invention aims to solve is to provide a method for reducing disability and/or mortality in resuscitated cardiac arrest patients; patients with ischemic or hemorrhagic stroke; patients with traumatic brain or spinal cord injury; or patients with hypoxic-ischemic encephalopathy (particularly infant patients), receiving targeted temperature management (TTM).

Another object that the present invention aims to solve is to provide a pharmaceutical composition for reducing disability and/or mortality in resuscitated cardiac arrest patients; patients with ischemic or hemorrhagic stroke; patients with traumatic brain or spinal cord injury; or patients with hypoxic-ischemic encephalopathy (particularly infant patients), receiving TTM treatment.

### Technical Solution

To achieve the above object, one aspect of the present invention provides a method for reducing mortality, improving neurological deficits, or improving daily living activities of a subject, characterized by administering a therapeutically effective amount of a compound of Chemical Formula 1 below or a pharmaceutically acceptable salt thereof to a subject who has suffered cardiac arrest and is undergoing targeted temperature management (TTM); a subject with ischemic or hemorrhagic stroke and is undergoing TTM; a subject with traumatic brain or spinal cord injury and is undergoing TTM; a subject with hypoxic-ischemic encephalopathy and is undergoing TTM; or a subject with hypoxic-ischemic encephalopathy and is undergoing therapeutic hypothermia.

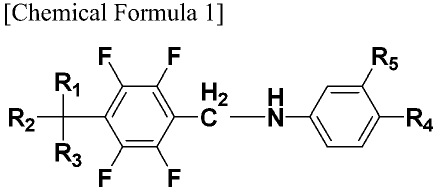

In Chemical Formula 1,
R₁, R₂, and R₃ are independently hydrogen or halogen,
R₄ is hydroxy, C₁-C₆ alkyl, C₁-C₆ alkoxy, halogen, C₁-C₁₀ alkanoyloxy, or nitro, and
R₅ is carboxylic acid, carboxylic acid ester, carboxamide, sulfonic acid, halogen, or nitro.

That is, one embodiment of the present invention provides a medical use of the compound of Chemical Formula 1 or a pharmaceutically acceptable salt thereof, wherein the compound of Chemical Formula 1 or a pharmaceutically acceptable salt thereof according to the present invention is useful for reducing mortality, improving neurological deficits, or improving activities of daily living in resuscitated cardiac arrest subjects receiving TTM; subjects with ischemic or hemorrhagic stroke receiving TTM; subjects with traumatic brain or spinal cord injury receiving TTM; subjects with hypoxic-ischemic encephalopathy receiving TTM; or subjects with hypoxic-ischemic encephalopathy receiving therapeutic hypothermia (particularly infants).

In one embodiment of the present invention, hypothermia treatment refers to a treatment method in which the core body temperature of a subject is maintained between 32°C and 36°C, and in particular, in a resuscitated cardiac arrest subject, hypothermia treatment refers to maintaining the core body temperature of the subject between 32°C and 36°C after successful resuscitation and the resumption of spontaneous circulation.

Accordingly, another embodiment of the present invention provides a pharmaceutical composition for reducing mortality, improving neurological deficits, or improving daily living activities in a resuscitated cardiac arrest subject receiving TTM; an ischemic or hemorrhagic stroke subject receiving TTM; a subject with traumatic brain or spinal cord injury receiving TTM; a subject with hypoxic-ischemic encephalopathy receiving TTM; or a subject (particularly infants) with hypoxic-ischemic encephalopathy receiving therapeutic hypothermia, comprising as an active ingredient a compound of Chemical Formula 1 or a pharmaceutically acceptable salt thereof.

The description of the embodiments of the present invention described below applies in common to all the methods, medical uses, and pharmaceutical compositions mentioned above.

In the present invention, the alkyl and alkoxy are each C₁-C₆ alkyl and C₁-C₆ alkoxy, for example, the alkyl is methyl, ethyl, propyl, isopropyl, n-butyl, sec-butyl, or tert-butyl, and the alkoxy means -O-(alkyl), including -OCH₃, -OCH₂CH₃, -O(CH₂)₂CH₃, -OC(CH₃)₂H, -OC(CH₃)₃, and similar ones. Preferably, the alkyl and alkoxy of the present invention are each C₁-C₃ alkyl and C₁-C₃ alkoxy.

In the present invention, the alkanoyloxy is C₁-C₁₀ alkanoyloxy, preferably C₂-C₁₀ alkanoyloxy, and more preferably C₃-C₈ alkanoyloxy. For example, the alkanoyloxy is ethanoyloxy, propanoyloxy, or cyclohexanecarbonyloxy.

In this specification, when described as "C₁-₆," "C1-6," or "C₁-C₆," it means that the number of carbon atoms is 1 to 6. For example, C₁-₆ alkyl means an alkyl having 1 to 6 carbon atoms.

In the present invention, "halogen" and "halo" mean fluorine, chlorine, bromine, or iodine. In a preferred embodiment of the present invention, the halogen is fluorine.

In the carboxylic acid ester of the present invention, the carbon may be substituted with methyl, ethyl, isopropyl, or butyl.

Pharmaceutically acceptable salts according to the present invention include ammonium salts; alkali metal salts such as lithium, sodium, or potassium; alkaline earth metal salts such as calcium or magnesium; salts of organic bases such as cyclohexylamine, benzylamine, octylamine, ethanolamine, diethylolamine, diethylamine, triethylamine, ethylenediamine, procaine, morpholine, pyrroline, piperidine, N-ethylpiperidine, N-methylmorpholine, piperazine, etc.; or salts of basic amino acids such as lysine, arginine, ornithine, histidine, etc.

Preferably, in the method, medical use, and pharmaceutical composition of the present invention, the subject is a subject who has suffered cardiac arrest and is receiving TTM. Patients who have been resuscitated after cardiac arrest have specific biological responses different from those of normal subjects, and when a compound according to the present invention is administered together with TTM, it is very useful for improving the patient's mortality rate, neurological deficits, and reduced daily life. In one embodiment of the present invention, the method, medical use, and pharmaceutical composition of the present invention can reduce white matter (brain nerve fiber) damage in resuscitated cardiac arrest patients receiving hypothermia treatment.

In another embodiment of the present invention, the method, medical use, and pharmaceutical composition of the present invention are also useful for treating patients with intracerebral hemorrhage, patients with subarachnoid hemorrhage, etc., who are receiving hypothermia treatment at 32-36°C.

Preferably, the compound of Chemical Formula 1 according to the present invention is
2-hydroxy-5-(2,3,5,6-tetrafluoro-4-trifluoromethylbenzylamino)benzoic acid (nelonemdaz),
2-nitro-5-(2,3,5,6-tetrafluoro-4-trifluoromethylbenzylamino)benzoic acid,
2-chloro-5-(2,3,5,6-tetrafluoro-4-trifluoromethylbenzylamino)benzoic acid,
2-bromo-5-(2,3,5,6-tetrafluoro-4-trifluoromethylbenzylamino)benzoic acid,
2-methyl-5-(2,3,5,6-tetrafluoro-4-trifluoromethylbenzylamino)benzoic acid,
2-methoxy-5-(2,3,5,6-tetrafluoro-4-trifluoromethylbenzylamino)benzoic acid,
5-(2,3,5,6-tetrafluoro-4-trifluoromethylbenzylamino)-2-trifluoromethoxybenzoic acid,
2-nitro-4-(2,3,5,6-tetrafluoro-4-trifluoromethylbenzylamino)phenol,
2-chloro-4-(2,3,5,6-tetrafluoro-4-trifluoromethylbenzylamino)phenol,
2-hydroxy-5-(2,3,5,6-tetrafluoro-4-trifluoromethylbenzylamino)benzamide,
2-hydroxy-5-(2,3,5,6-tetrafluoro-4-trifluoromethylbenzylamino)benzenesulfonic acid,
methyl 2-hydroxy-5-(2,3,5,6-tetrafluoro-4-trifluoromethylbenzylamino)benzoate,
2-ethanoyloxy-5-(2,3,5,6-tetrafluoro-4-trifluoromethylbenzylamino)benzoic acid,
2-propanoyloxy-5-(2,3,5,6-tetrafluoro-4-trifluoromethylbenzylamino)benzoic acid,
2-cyclohexanecarbonyloxy-5-(2,3,5,6-tetrafluoro-4-trifluoromethylbenzylamino)benzoic acid, or pharmaceutically acceptable salts thereof.

More preferably, the compound of Chemical Formula 1 according to the present invention is 2-hydroxy-5-(2,3,5,6-tetrafluoro-4-trifluoromethylbenzylamino)benzoic acid (nelonemdaz) or a pharmaceutically acceptable salt thereof.

Even more preferably, the compound of Chemical Formula 1 according to the present invention is potassium salt of 2-hydroxy-5-(2,3,5,6-tetrafluoro-4-trifluoromethylbenzylamino)benzoic acid.

The compound of Chemical Formula 1 or a salt thereof according to the present invention may be prepared by the reaction scheme set forth in U.S. Patent No. 7,511,074, but is not limited thereto.

One embodiment of the present invention also provides a composition comprising a compound of Chemical Formula 1 or a pharmaceutically acceptable salt thereof; and a pharmaceutically acceptable carrier or additive. That is, in one embodiment of the present invention, the compound of Chemical Formula 1 or a salt thereof is administered together with a carrier, additive, etc., which are commonly used in the field to which the present invention belongs. However, the compound of Chemical Formula 1 or a salt thereof of the present invention may be administered alone.

In one embodiment of the present invention, the composition according to the present invention comprises about 50 mg to about 2,000 mg, preferably about 250 mg to about 2,000 mg of the compound of Chemical Formula 1. In another embodiment, the composition of the present invention comprises about 100 mg to about 1,000 mg of the compound of Chemical Formula 1. In yet another embodiment, the composition of the present invention comprises about 50 mg to about 500 mg of the compound of Chemical Formula 1.

In one embodiment of the present invention, the formulation (composition) of the present invention for administration may be a single-dose unit or a multi-dose unit. In some embodiments, the formulation (composition) of the present invention is a single-dose unit. In other embodiments, the formulation (composition) of the present invention is a multi-dose unit and is divided for use in multiple administrations.

The pharmaceutical composition of the present invention may be administered in the form of an injectable formulation (e.g., intramuscular, intraperitoneal, intravenous, infusion, subcutaneous, implant), but is not limited thereto. Depending on the route of administration, the pharmaceutical composition of the present invention may be formulated into appropriate dosage units comprising pharmaceutically acceptable and non-toxic carriers, additives, and/or vehicles commonly used in the art. Additionally, sustained-release formulations capable of continuously releasing the drug over a target time are included within the scope of the present invention.

In a preferred embodiment according to the present invention, 50 mg to 2000 mg of the compound of Chemical Formula 1 or a pharmaceutically acceptable salt is contained in sterile vial(s), said composition is dissolved in nanopure water in the pH range of 8 to 11, filtered, and freeze-dried under conditions filled with nitrogen gas. All formulation processes are carried out under conditions filled with nitrogen gas. The composition contained in the vial is redissolved in sterile water for infusion and further diluted with 0.9% physiological saline before infusion to a human. Two additional freeze-drying steps are carried out until a nearly white fine cake without needle-shaped crystals is obtained. In one embodiment of the present invention, the compound according to the present invention is administered as an injectable solution containing 50 mg to 2000 mg, preferably with a pH of 8-11.

In one embodiment of the present invention, 50 to 2000 mg of the compound of Chemical Formula 1 or a pharmaceutically acceptable salt thereof is contained in sterile vial(s), said composition is dissolved in tris(hydroxymethyl)aminomethane [THAM]-buffered water for injection, filtered, and lyophilized under nitrogen gas-filled conditions. The composition contained in the vial is redissolved in water for injection for injection and further diluted with 0.9% physiological saline before injection into a person.

In another embodiment of the present invention, 50 to 2000 mg of the compound of Chemical Formula 1 or a pharmaceutically acceptable salt thereof is contained in sterile vial(s), said composition is dissolved in tris(hydroxymethyl)aminomethane [THAM]-buffered water for injection, filtered, and lyophilized under nitrogen gas-filled conditions. The composition contained in the vial is redissolved in THAM buffer for injection and further diluted with 0.9% physiological saline before injection into a person.

In one embodiment of the present invention, the compound of Chemical Formula 1 or a pharmaceutically acceptable salt thereof is administered to a subject in a dose of 100 mg to 4000 mg daily for the aforementioned use. In another embodiment, particularly 2-hydroxy-5-[2-(4-trifluoromethyl-phenyl)-ethylamino]-benzoate potassium salt ('nelonemdaz K') is administered to a person in a dose of 250 mg to 3000 mg daily for 1 to 3 days. In another embodiment, nelonemdaz K is administered intravenously (IV) to the aforementioned patients receiving TTM according to the following administration schedule, particularly to resuscitated cardiac arrest patients:
(1) For a total dose of 6,000 mg nelonemdaz K: 1,500 mg at the first dose, 1,500 mg at 12 ± 6 hour intervals at the second dose, and 750 mg at 12 ± 1 hour intervals at the 3^{rd} to 6^{th} infusions;
(2) For a total dose of 5,250 mg nelonemdaz K: 1,500 mg at the first dose, 750 mg at 12 ± 6 hour intervals at the second dose, and 750 mg at 12 ± 1 hour intervals at the 3^{rd} to 6^{th} infusions;
(3) For a total dose of 4,250 mg nelonemdaz K: 1,500 mg at the first dose, 750 mg at 12 ± 6 hour intervals at the second dose, and 500 mg at 12 ± 1 hour intervals at the 3^{rd} to 6^{th} infusions; or
(4) For a total dose of 3,250 mg nelonemdaz K: 750 mg at the first dose, 500 mg at 12 ± 6 hour intervals at the second dose, and 500 mg at 12 ± 1 hour intervals at the 3^{rd} to 6^{th} infusions.

In another embodiment of the present invention, nelonemdaz K is administered by intravenous injection (IV) to the aforementioned patients receiving TTM according to the following administration schedule, in particular, to resuscitated cardiac arrest patients:
(a) administered at a dose of 250 mg to 1500 mg twice daily for 1 to 3 days;
(b) administered a total dose of 5,250 mg over 3 days;
(c) administered a total of 6 times over 3 days, with the first dose being 1,500 mg and the remaining doses being 750 mg each, with each dose administered at approximately 12-hour intervals;
(d) administered a total dose of 3,250 mg over 3 days; or
(e) administered a total of 6 times over 3 days, with the first dose being 750 mg and the remaining doses being 500 mg each, with each dose administered at approximately 12-hour intervals.

However, the treatment method (dose, treatment route, interval, type of infusion, etc.) of the compound of the present invention or its salt is not limited to the method described above.

In one embodiment of the present invention, the compounds and compositions of the present invention are administered via an intravenous route, either by direct intravenous injection or, in the case of a patient receiving an intravenous infusion, by mixing with the composition during the infusion.

In one embodiment of the present invention, the compound of Chemical Formula 1 according to the present invention or a pharmaceutically acceptable salt thereof may be administered together with an antioxidant to enhance the effect of hypothermia treatment. In another embodiment, to enhance the effect of hypothermia treatment, the compound of Chemical Formula 1 according to the present invention or a pharmaceutically acceptable salt thereof may be administered first, and an antioxidant may be administered secondarily.

The compound of Chemical Formula 1 according to the present invention or a pharmaceutically acceptable salt thereof may be used as follows.

### Application Example 1: Cardiac Arrest Patients Receiving Hypothermia Treatment

Sudden cardiac arrest is one of the leading causes of death and disease worldwide, accounting for half of all deaths due to cardiovascular disease. Brain damage occurring after total cerebral ischemia-reperfusion is a cause of disability and death due to cardiac arrest. Currently, hypothermia treatment is recommended as a guideline to reduce ischemia-reperfusion injury occurring in resuscitated cardiac arrest patients. As described above, the present invention provides evidence that nelonemdaz has an additional therapeutic effect on cardiac arrest patients who have been resuscitated and are receiving hypothermia treatment.

### Application Example 2: Stroke Patients Undergoing Hypothermia Treatment

The Neuroprotective Therapy Consensus Review (NTRC) group announced that it recommends hypothermia therapy for patients with intracerebral hemorrhage, aneurysmic subarachnoid hemorrhage, and acute ischemic stroke. Stroke occurs when cerebral blood flow is blocked by blood clots, embolic substances, or the rupture of blood vessels; it is a major disease that causes severe long-term disability in adults and is the second leading cause of death in the United States and Europe. Neuronal death following a stroke leads to permanent disability and death. Hypothermia therapy provides neuroprotective effects through various physiological mechanisms in animal models of ischemic and hemorrhagic stroke as well as in stroke patients. Nelonemdaz can be used adjunctively to reduce brain damage in patients with intracerebral hemorrhage, aneurysmic subarachnoid hemorrhage, and acute ischemic stroke undergoing hypothermia therapy.

### Application Example 3: Patients with Traumatic Brain Injury and Spinal Cord Injury Receiving Hypothermia Treatment

Traumatic brain injury (TBI) is a serious problem, causing disability and death to 69 million people worldwide every year. In animal models, brain edema and neuronal death caused by TBI are reported to be reduced by hypothermia treatment, and disability is also reported to improve. Beneficial effects of hypothermia treatment have also been reported in TBI patients. Furthermore, the neuroprotective and disability-improving effects of hypothermia treatment have been proven in animal models and clinical trials for traumatic spinal cord injury (TSCI). Therefore, nelonemdaz can be used to additionally reduce brain damage and disability in TBI and TSCI patients receiving hypothermia treatment.

### Application Example 4: Hypoxic-Ischemic Encephalopathy in Neonates Receiving Hypothermia Treatment

Neonatal hypoxic-ischemic encephalopathy (HIE) is a severe birth complication in which brain nerve tissue is damaged due to a lack of oxygen and blood supply, affecting approximately 400,000 newborns worldwide annually. Hypothermia is the only approved standard treatment to reduce brain damage in newborns suffering from HIE. However, HIE still accounts for about one-fifth of neonatal deaths worldwide. Hypothermia can reduce neuronal death by preventing moderate to severe hypoxic-ischemic encephalopathy in newborns. nelonemdaz can be used as an adjunct to HIE patients undergoing hypothermia to protect brain cells and further reduce disability and mortality.

### Advantageous Effects

The compound of Chemical Formula 1 according to the present invention or a pharmaceutically acceptable salt thereof may be used to reduce mortality, improve neurological deficits, or improve daily living activities by additionally administering it to patients with cardiac arrest, patients with ischemic or hemorrhagic stroke, patients with traumatic brain and spinal cord injury, or patients suffering from hypoxic and ischemic encephalopathy (particularly newborns or infants), receiving hypothermia treatment.

### DESCRIPTION OF DRAWINGS

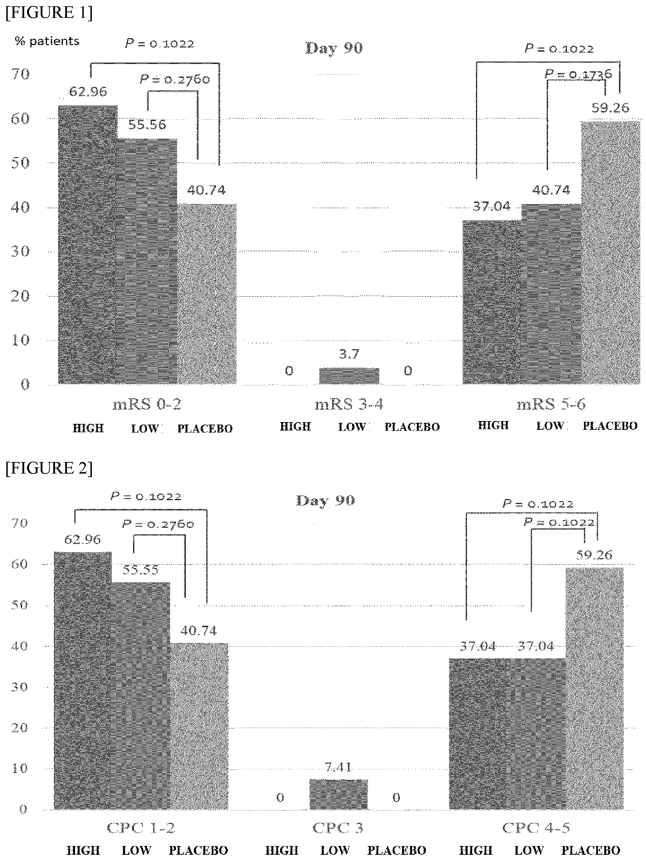

Figure 1 is a graph showing the proportion of cardiac arrest patients who successfully resuscitated and received hypothermia treatment with mRS scores of 0-2, 3-4, and 5-6 on day 90 after the first administration of the drug.
   mRS 0-2, patients capable of independent activity (Good neurological outcome)
   mRS 3-4, patients with moderate disability
   mRS 5-6, patients with severe disability (coma) or death (Poor neurological outcome)
   HIGH, high-dose nelonemdaz treatment group; LOW, low-dose nelonemdaz treatment group; PLACEBO, placebo treatment group
Figure 2 is a graph showing the proportion of cardiac arrest patients who successfully resuscitated and received hypothermia treatment with CPC scores of 1-2, 3, and 4-5 on day 90 after the first administration of the drug.
   CPC 1-2, patients capable of independent activity (Good neurological outcome)
   CPC 3, patients with moderate disability
   CPC 4-5, patients in coma or brain death (Poor neurological outcome)
   HIGH, high-dose nelonemdaz group; LOW, low-dose nelonemdaz group; PLACEBO, placebo group

### MODE FOR DISCLOSURE

Hereinafter, the present invention will be described in detail with reference to examples and the like to aid in understanding the invention. However, the examples according to the present invention may be modified in various different forms, and the scope of the present invention should not be interpreted as being limited to the following examples. The examples of the present invention are provided to more completely explain the invention to those with average knowledge in the field to which the invention pertains.

### 1. Cardiac arrest clinical trial phase 2 (AWAKE) trial design

The Phase 2 study (AWAKE) was conducted in patients with severe or comatose cardiac arrest who returned to spontaneous circulation and arrived at the emergency department within 4 hours and received therapeutic hypothermia (TTM). This study was conducted to investigate the efficacy and safety of nelonemdaz administered intravenously to subjects for 3 days. A total of 105 patients aged 19 to 80 years were enrolled in the AWAKE trial and were randomly assigned in a double-blind fashion to one of three groups to receive intravenous administration of the test drug. The placebo group received 250 ml of 0.9% saline intravenously six times over three days at 12-hour intervals. The low-dose group received a total of 3,250 mg of nelonemdaz (750 mg for the first dose, then 500 mg every 12 hours for the second to sixth doses). The high-dose group received a total of 5,250 mg of nelonemdaz (1,500 mg for the first dose, then 750 mg every 12 hours for the second to sixth doses). Among patients with cardiac arrest outside the hospital, those whose spontaneous circulation resumed for more than 20 minutes and who received hypothermia treatment at 32-34°C for 24 hours upon arrival at the hospital were enrolled. Patients were administered the test drug within 4 hours after spontaneous circulation resumed. Neurological outcomes were analyzed using the modified Rankin Scale (mRS) and brain performance category (CPC) analysis at 90 days after the first dose for patients in a severe or coma state before administration of the test drug, and the degree of brain white matter damage was evaluated using diffusion tensor MRI (DTI) images on the 4^{th} or 5^{th} day after the first dose.

### Modified Rankin Scale (mRS)

The Modified Rankin Scale is an analytical method widely used in clinical trials to assess the degree of disability in patients with stroke and cardiac arrest. Evaluation was performed based on the criteria shown in the table below.

**[Table 1]**

| **mRS Score** | **Assessment Criteria** |
|---|---|
| 0 | No disability |
| 1 | Symptomatic but no disability |
| 2 | Mild disability allowing independent activities |
| 3 | Mild disability requiring assistance but able to walk |
| 4 | Severe disability unable to walk but able to eat and wash independently |
| 5 | Severe disability requiring constant assistance and confined to bed |
| 6 | Death |

### Cerebral Performance Category (CPC)

The Cerebral Performance Category (CPC) is used as a scale to analyze neurological outcomes after cardiac arrest, with a score ranging from 0 (normal) to 5 (dead). It was evaluated based on the criteria shown in the table below.

**[Table 2]**

| **CPC Score** | **Assessment Criteria** |
|---|---|
| **1** | Normal, symptoms may be present but no disability |
| **2** | Moderate disability, disability present but able to perform independent activities |
| **3** | Severe disability, conscious but requires assistance with daily activities |
| **4** | Coma, unconscious |
| **5** | Brain death |

### Diffusion Tensor MR Imaging (DTI)

In brain white matter, the diffusion of water molecules moves in a direction parallel to the direction of nerve fiber bundles. Fractional anisotropy (FA) indicates the direction of water molecule diffusion, and low anisotropy implies that the directionality of water diffusion is reduced due to damage to the brain white matter. The protective effect of nelonemdaz on brain white matter was analyzed by comparing whole-brain and region-specific anisotropy values obtained from brain DTI images of resuscitated cardiac arrest patients who received nelonemdaz with those of a placebo group. Resuscitated cardiac arrest patients receive TTM treatment upon arrival at the hospital and are administered medication for three days within four hours of resuscitation. Diffusion tensor MRI images were taken within 48 hours of the last medication administration.

To analyze FA values in the whole brain tissue, preprocessing such as image and motion noise removal and skull removal was performed to extract diffusion parameters, and FA values corresponding to the whole brain tissue were calculated.

To calculate the average FA for each region, individual FA maps were translated and aligned into a standardized space using non-linear registration techniques. Since FA primarily identifies the white matter region, calculations were performed by overlaying only the JHU DTI-based white-matter atlas.

The Wilcoxon rank sum test was used to analyze the statistical differences between the high-dose group and the placebo control group, as well as between the low-dose group and the placebo control group.

### Experimental Example 1: Safety of Nelonemdaz in Cardiac Arrest Patients Who Received Hypothermia Treatment After Successful Resuscitation

Unexpected adverse effects, such as symptoms of schizophrenia, have been observed in healthy individuals and ischemic stroke patients treated with NMDA receptor antagonists, which has limited their clinical studies and applications (Hoyte, L. et al., (2004). "The rise and fall of NMDA antagonists for ischemic stroke." Curr Mol Med 4(2): 131-136.). The safety of nelonemdaz, a selective NR2B NMDA receptor antagonist, was investigated in cardiac arrest patients who received hypothermia treatment after successful resuscitation. The results are shown in the table below.

Summary of Serious Adverse Events (AEs) Occurring After Administration of Placebo and nelonemdaz-K in Cardiac Arrest Patients Who Received Hypothermia Treatment After Successful Resuscitation

**[Table 3]**

| **Safety** | | **Nelonemdaz K 5.25g (N=37)** | **Nelonemdaz K 3.25g (N=34)** | **Placebo (N=33)** |
|---|---|---|---|---|
| Frequency of serious adverse events | N | 0 (0.00%) | 0 (0.00%) | 0 (0.00%) |
| Frequency of medication-related serious adverse events | N | 0 (0.00%) | 0 (0.00%) | 0 (0.00%) |
| Remarks: Number of patients who experienced the event (% of patients who experienced the event) | | | | |

Compared to the placebo group, no unusual side effects, including schizophrenia symptoms, were observed in the low-dose (3,250 mg) or high-dose nelonemdaz (5,250 mg) group.

### Experimental Example 2: Disability Improvement Effect of Nelonemdaz in Cardiac Arrest Patients Resuscitated and Receiving Hypothermia Treatment (mRS Assessment)

The disability improvement efficacy of nelonemdaz was analyzed using the modified Rankin Scale (mRS) in cardiac arrest patients resuscitated and receiving TTM treatment. On day 90 after the first drug administration, the proportion of patients with an mRS of 0 (normal) in the placebo group was 37.04%, while the proportion of patients with an mRS of 0 in the low-dose and high-dose nelonemdaz groups increased to 48.15% and 55.56%, respectively. Detailed results are shown in the table below.

Modified Rankin Scale (mRS) scores before drug administration and on day 90 after the first administration in resuscitated cardiac arrest patients receiving hypothermia treatment

**[Table 4]**

| | | **Nelonemdaz K 5.25g (N=27)** | **Nelonemdaz K 3.25g (N=27)** | **Placebo (N=27)** |
|---|---|---|---|---|
| Before administration | | N=27 | N=27 | N=27 |
| mRS | 0 | 0 | 0 | |
| | 1 | 0 | 0 | |
| | 2 | 0 | 0 | |
| | 3 | 0 | 0 | |
| | 4 | 0 | 0 | 3 (10.34%) |
| | 5 | 27 (100%) | 27 (100%) | 24 (82.76%) |
| | 6 | 0 | 0 | 0 |

| On day 90 after the first drug administration | | N=27 | N=27 | N=27 |
|---|---|---|---|---|
| mRS | 0 | 15 (55.56%) | 13 (48.15%) | 10 (37.04%) |
| | 1 | 1 (3.70%) | 2 (7.41%) | 1 (3.70%) |
| | 2 | 1 (3.70%) | 0 (0.00%) | 0 (0.00%) |
| | 3 | 0 (0.00%) | 1 (3.70%) | 0 (0.00%) |
| | 4 | 0 (0.00%) | 0 (0.00%) | 0 (0.00%) |
| | 5 | 1 (3.70%) | 3 (11.11%) | 2 (7.41%) |
| | 6 | 9 (33.33%) | 8 (29.63%) | 14 (51.85%) |
| Common Odds Ratio* (95% C.I.) | | 2.243 (0.806, 6.242) | 1.975 (0.714, 5.461) | 1 (Ref) |

| | | | | |
|---|---|---|---|---|
| * Odds ratio (95% CI) of shift in the distribution of mRS scores showing improvement in disability in the nelonemdaz treatment group compared to the placebo group | | | | |

On day 90 after drug administration, a distinct trend of reduced disability and mortality was observed in patients treated with nelonemdaz-K compared to the placebo group. In particular, the proportion of subjects with an mRS of 0-2 (good neurological outcome: patients capable of independent activity) increased 1.375-fold from 40.74% (11/27) in the placebo group to 55.56% (15/27) in the low-dose group, while the high-dose group improved 1.55-fold to 62.96% (17/27) (Figure 1). On day 90 after drug administration, the proportion of subjects with an mRS of 5-6 (poor neurological outcome: severe coma or death) decreased 1.31-fold from 59.26% (16/27) in the placebo group to 40.74% (11/27) in the low-dose group, while the high-dose group improved 1.38-fold to 37% (10/27).

These results suggest that in cardiac arrest patients who are successfully resuscitated and are in a coma, receiving combination therapy with nelonemdaz and TTM within 4 hours improves disability and reduces coma and death.

### Experimental Example 3: Effect of Nelonemdaz on Improving Disability in Cardiac Arrest Patients Resuscitated and Receiving Hypothermia Treatment (CPC Assessment)

Meanwhile, using the Cerebral Performance Category (CPC; 1 = Normal, 2 = Moderate Impairment, 3 = Severe Impairment, 4 = Coma, 5 = Death), the effect of nelonemdaz was analyzed in cardiac arrest patients with severe impairment or coma who had successfully resuscitated and were receiving hypothermia treatment. The results are shown in the table below.

Cerebral Performance Category (CPC) scores before drug administration and on day 90 after the first administration to cardiac arrest patients who had resuscitated and received hypothermia treatment

**[Table 5]**

| | | **Nelonemdaz K 5.25g (N=27)** | **Nelonemdaz K 3.25g (N=27)** | **Placebo (N=27)** |
|---|---|---|---|---|
| | N | 27 | 27 | 27 |
| Before administration | CPC 1 | 0 (0.00%) | 0 (0.00%) | 0 (0.00%) |
| | CPC 2 | 0 (0.00%) | 0 (0.00%) | 0 (0.00%) |
| | CPC 3 | 1 (3.70%) | 1 (3.70%) | 1 (3.70%) |
| | CPC 4 | 26 (96.30%) | 26 (96.30%) | 26 (96.30%) |
| | CPC 5 | 0 (0.00%) | 0 (0.00%) | 0 (0.00%) |
| | Common Odds Ratio* (95% C.I.) | 1.00(0.06-16.86) | 1.00 (0.06-16.86) | 1(Ref) |

| | N | 27 | 27 | 27 |
|---|---|---|---|---|
| On day 90 after the first drug administration | CPC 1 | 16 (59.26%) | 15 (55.56%) | 11 (40.74%) |
| | CPC 2 | 1 (3.70%) | 0 (0.00%) | 0 (0.00%) |
| | CPC 3 | 0 (0.00%) | 2 (7.41%) | 0 (0.00%) |
| | CPC 4 | 1 (3.70%) | 2 (7.41%) | 2 (7.41%) |
| | CPC 5 | 9 (33.33%) | 8 (29.63%) | 14 (51.85%) |
| | Common Odds Ratio* (95% C.I.) | 2.22 (0.78-6.30) | 2.13 (0.75-6.03) | 1 (Ref) |

| | | | | |
|---|---|---|---|---|
| *: Odds ratio (95% CI) of the shift in the distribution of CPC scores showing disability improvement in the nelonemdaz treatment group compared to the placebo group | | | | |

As shown in the table above, on day 90 after the first drug administration, the proportion of patients who completely recovered to CPC 1 in the placebo group was 40.74%, while it increased to 55.56% and 59.26% in the low-dose nelonemdaz group and the high-dose nelonemdaz group, respectively. Additionally, the proportion of patients with CPC 5 who died was found to decrease in the low-dose (29.63%) and high-dose (33.33%) nelonemdaz groups compared to the placebo group (51.85%).

The proportion of patients with the CPC 1-2 (Good Outcome) who recovered to the point of independent living on day 90 after drug administration was 40.74% in the placebo group, increased 1.36-fold to 55.55% in the low-dose nelonemdaz group, and improved 1.55-fold to 62.96% in the high-dose nelonemdaz group (Figure 2). The proportion of patients with the CPC 4-5 (Poor Outcome) who deteriorated into a coma or brain death was 59.26% in the placebo group, which decreased to 37.04% in the low-dose and high-dose nelonemdaz groups. The results of the CPC analysis suggest that in cardiac arrest patients who successfully resuscitate and receive hypothermia treatment, the administration of nelonemdaz improves disability and reduces coma and brain death.

### Experimental Example 4: Brain Tissue Protective Effect of Nelonemdaz in Cardiac Arrest Patients Resuscitated and Receiving Hypothermia Treatment

The effect of nelonemdaz on brain white matter (nerve fibers including axons) damage was investigated by comparing fractional anisotropy (FA) values obtained from Diffusion Tensor Magnetic Resonance Imaging (DTI, Diffusion Tensor MRI) in cardiac arrest patients who were resuscitated and received TTM treatment. DTI is a method that assesses the level of brain damage by utilizing diffusion information of water molecules between nerve fiber bundles constituting the white matter within brain tissue; while normal cerebral white matter exhibits high diffusion anisotropy, the presence of brain damage results in reduced diffusion anisotropy. Damage to the entire brain and by region was analyzed by comparing FA (fractional anisotropy), which can quantitatively determine this diffusion anisotropy.

Diffusion tensor imaging (DTI) was performed on 54 patients out of 105 randomly assigned according to the clinical protocol, including 14 in the placebo group, 22 in the low-dose nelonemdaz group, and 18 in the high-dose nelonemdaz group; white matter damage was analyzed in patients for whom imaging was available. The results are shown in the table below.

Fractional anisotropy (FA) of whole brain white matter and by region on day 5 after the first drug administration in cardiac arrest patients who successfully resuscitated and received hypothermia treatment

**[Table 6]**

| | | HIGH Dose Nelonemdaz (n=18) | LOW Dose Nelonemdaz (n = 22) | Placebo (n = 14) | p-value, HIGH versus Placebo | p-value, LOW versus Placebo |
|---|---|---|---|---|---|---|
| Fractional anisotropy (FA) | | Median (interquartile range) | | | | |
| Whole brain white matter | | 0·465 (0·449-0·485) | 0·462 (0·439-0·480) | 0·441(0·431-0·464) | 0·028 | 0·19 |

| White matter by region | | | | | | |
|---|---|---|---|---|---|---|
| | Corpus callosum | 0·581 (0·564-0·602) | 0·565 (0·534-0·600) | 0·538 (0·531-0·563) | 0·004 | 0·20 |
| | Cingulum (cingulate gyrus) | 0·419 (0·400-0·448) | 0·411 (0·397-0·431) | 0·395 (0·389-0·408) | 0·06 | 0·07 |
| | Cingulum (hippocampus) | 0·329 (0·308-0·353) | 0·317 (0·289-0·340) | 0·310 (0·297-0·342) | 0·30 | 0·74 |
| | Superior longitudinal fasciculus | 0·400 (0·384-0·411) | 0·414 (0·397-0·421) | 0·402 (0·391-0·418) | 0·64 | 0·55 |
| | Inferior fronto-occipital fasciculus | 0·420 (0·400-0·446) | 0·400 (0·370-0·429) | 0·384 (0·369-0·441) | 0·08 | 0·86 |
| | Uncinate fasciculus | 0·312 (0·297-0·322) | 0·285 (0·266-0·341) | 0·281 (0·274-0·301) | 0·045 | 0·89 |
| | Superior fronto-occipital fasciculus | 0·400 (0·391-0·429) | 0·355 (0·340-0·407) | 0·338 (0·310-0·384) | 0·016 | 0·21 |
| | Fornix | 0·440 (0·414-0·450) | 0·405 (0·369-0·435) | 0·390 (0·361-0·425) | 0·006 | 0·43 |
| | Anterior limb of internal capsule | 0·470 (0·449-0·498) | 0·464 (0·437-0·483) | 0·436 (0·422-0·468) | 0·020 | 0·15 |
| | Posterior limb of internal capsule | 0·581 (0·566-0·598) | 0·578 (0·552-0·596) | 0·567 (0·552-0·595) | 0·18 | 0·49 |
| | Retrolenticular internal capsule | 0·502 (0·487-0·535) | 0·504 (0·490-0·527) | 0·494 (0·460-0·520) | 0·25 | 0·24 |
| | External capsule | 0·368 (0·351-0·372) | 0·359 (0·328-0·376) | 0·349 (0·321-0·362) | 0·13 | 0·39 |
| | Sagittal stratum | 0·441 (0·427-0·447) | 0·430 (0·423-0·445) | 0·425 (0·410-0·433) | 0·11 | 0·31 |
| | Superior corona radiata | 0·434 (0·413-0·454) | 0·431 (0·393-0·462) | 0·427 (0·413-0·438) | 0·42 | 0·64 |
| | Posterior corona radiata | 0·422 (0·409-0·456) | 0·420 (0·391-0·443) | 0·414 (0·390-0·428) | 0·14 | 0·64 |
| | Anterior corona radiata | 0·372 (0·357-0·382) | 0·358 (0·338-0·390) | 0·347 (0·324-0·372) | 0·05 | 0·24 |
| | Middle cerebellar peduncle | 0·434 (0·416-0·443) | 0·418 (0·408-0·448) | 0·415 (0·401-0·435) | 0·022 | 0·33 |
| | Inferior cerebellar peduncle | 0·412 (0·395-0·421) | 0·407 (0·390-0·429) | 0·409 (0·372-0·426) | 0·78 | 0·45 |
| | Superior cerebellar peduncle | 0·505 (0·499-0·526) | 0·484 (0·471-0·506) | 0·486 (0·456-0·504) | 0·034 | 0·60 |
| | Pontine crossing tract | 0·414 (0·400-0·450) | 0·412 (0·394-0·423) | 0·412 (0·377-0·431) | 0·28 | 0·64 |
| | Corticospinal tract | 0·445 (0·428-0·466) | 0·448 (0·431-0·470) | 0·436 (0·426-0·469) | 0·78 | 0·31 |
| | Medial lemniscus | 0·517 (0·506-0·533) | 0·517 (0·493-0·539) | 0·515 (0·494-0·544) | 0·59 | 0·94 |
| | Posterior thalamic radiation | 0·496 (0·482-0·519) | 0·489 (0·465-0·515) | 0·481 (0·450-0·503) | 0·13 | 0·45 |
| | Cerebral peduncle | 0·587 (0·574-0·602) | 0·582 (0·559-0·610) | 0·569 (0·557-0·593) | 0·10 | 0·18 |

The median total white matter FA was 0.441 (0.431-0.464) in the placebo group, increased to 0.462 (0.439-0.480) in the low-dose nelonemdaz group, and 0.465 (0.449-0.485) in the high-dose nelonemdaz group. In particular, the total white matter FA value increased significantly (P = 0.028) in the high-dose nelonemdaz group compared to the placebo group.

A general increasing trend was observed in the median FA values by region in the high-dose nelonemdaz group compared to the placebo group. In particular, damage to the corpus callosum (connecting the left and right hemispheres), superior fronto-occipital fasciculus (connecting the frontal and occipital lobes), fornix (connecting the limbic system responsible for cognitive function and memory), anterior limb of internal capsule (connecting the cerebral cortex and thalamus), middle cerebellar peduncle (connecting the pons and cerebellum), and superior cerebellar peduncle (connecting the midbrain and cerebellum) was significantly reduced by the administration of high doses of nelonemdaz.

White matter accounts for 50% of the total brain and is known to be susceptible to damage even from brief, transient cerebral ischemia. Generalized white matter damage is observed in the brains of resuscitated cardiac arrest patients, and DTI imaging taken within three weeks of cardiac arrest has reported that white matter damage is greater (lower FA values) in patients with a poor prognosis compared to those with a good prognosis. These research results indicate that the initial white matter protective effect of nelonemdaz, verified in resuscitated cardiac arrest patients, is associated with long-term improvement in neurological function.

## Claims

1. A method for reducing mortality, improving neurological deficits, or improving daily living activities in a subject, **characterized by** administering a therapeutically effective amount of a compound of Chemical Formula 1 below or a pharmaceutically acceptable salt thereof to a subject who has suffered cardiac arrest and is receiving targeted temperature management (TTM); a subject with ischemic or hemorrhagic stroke and is receiving TTM; a subject with traumatic brain or spinal cord injury and is receiving TTM; a subject with hypoxic-ischemic encephalopathy and is receiving TTM; or a subject with hypoxic-ischemic encephalopathy and is receiving therapeutic hypothermia: 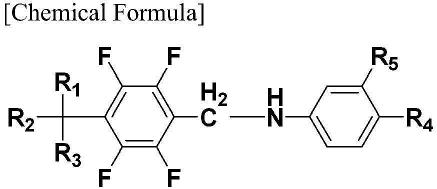 In Chemical Formula 1,
R₁, R₂, and R₃ are independently hydrogen or halogen,
R₄ is hydroxy, C₁-C₆ alkyl, C₁-C₆ alkoxy, halogen, C₁-C₁₀ alkanoyloxy, or nitro, and
R₅ is carboxylic acid, carboxylic acid ester, carboxamide, sulfonic acid, halogen, or nitro.

2. The method of claim 1, wherein the subject is a subject that has undergone cardiac arrest and is receiving TTM.

3. The method of claim 1 or 2, wherein the compound is any selected from the group consisting of
2-hydroxy-5-(2,3,5,6-tetrafluoro-4-trifluoromethylbenzylamino)benzoic acid (nelonemdaz),
2-nitro-5-(2,3,5,6-tetrafluoro-4-trifluoromethylbenzylamino)benzoic acid,
2-chloro-5-(2,3,5,6-tetrafluoro-4-trifluoromethylbenzylamino)benzoic acid,
2-bromo-5-(2,3,5,6-tetrafluoro-4-trifluoromethylbenzylamino)benzoic acid,
2-methyl-5-(2,3,5,6-tetrafluoro-4-trifluoromethylbenzylamino)benzoic acid,
2-methoxy-5-(2,3,5,6-tetrafluoro-4-trifluoromethylbenzylamino)benzoic acid,
5-(2,3,5,6-tetrafluoro-4-trifluoromethylbenzylamino)-2-trifluoromethoxybenzoic acid,
2-nitro-4-(2,3,5,6-tetrafluoro-4-trifluoromethylbenzylamino)phenol,
2-chloro-4-(2,3,5,6-tetrafluoro-4-trifluoromethylbenzylamino)phenol,
2-hydroxy-5-(2,3,5,6-tetrafluoro-4-trifluoromethylbenzylamino)benzamide,
2-hydroxy-5-(2,3,5,6-tetrafluoro-4-trifluoromethylbenzylamino)benzenesulfonic acid,
methyl 2-hydroxy-5-(2,3,5,6-tetrafluoro-4-trifluoromethylbenzylamino)benzoate,
2-ethanoyloxy-5-(2,3,5,6-tetrafluoro-4-trifluoromethylbenzylamino)benzoic acid,
2-propanoyloxy-5-(2,3,5,6-tetrafluoro-4-trifluoromethylbenzylamino)benzoic acid, and
2-cyclohexanecarbonyloxy-5-(2,3,5,6-tetrafluoro-4-trifluoromethylbenzylamino)benzoic acid.

4. The method of claim 1, wherein the compound is 2-hydroxy-5-(2,3,5,6-tetrafluoro-4-trifluoromethylbenzylamino)benzoic acid or a pharmaceutically acceptable salt thereof.

5. The method of claim 1, wherein the compound is administered as a potassium salt of 2-hydroxy-5-(2,3,5,6-tetrafluoro-4-trifluoromethylbenzylamino)benzoic acid.

6. The method of claim 1 or 2, wherein the compound is administered as an injectable liquid containing 50 mg to 2000 mg of the compound.

7. The method of claim 6, wherein the pH of the injectable liquid is 8-11.

8. The method of claim 1 or 2, wherein the compound is **characterized by**:
(a) administered at a dose of 250 mg to 1500 mg twice a day for 1 to 3 days;
(b) administered at a total dose of 5,250 mg for 3 days;
(c) administered at a total of 6 doses for 3 days, wherein the first dose is 1500 mg and the remaining doses are each 750 mg, with each dose administered at approximately 12-hour intervals;
(d) administered at a total dose of 3,250 mg for 3 days; or
(e) administered at a total of 6 doses for 3 days, wherein the first dose is 750 mg and the remaining doses are each 500 mg, with each dose administered at approximately 12-hour intervals.

9. The method of claim 8, wherein the administration is performed via an intravenous route.

10. The method of claim 1 or 2, wherein the hypothermia treatment is performed by maintaining the subject's core body temperature between 32°C and 36°C.
